# EUROPEAN PATENT APPLICATION

(11) **EP 4 692 114 A1**
(43) Date of publication of application: **11.02.2026**
(21) Application number: 24784359.2
(22) Date of filing: 03.04.2024
(51) Int. Cl.: C07K 14/585, A61K 38/23

(54) **CALCITONIN ANALOGUE, AND DERIVATIVE THEREOF AND USE THEREOF**

(30) Priority: 04.04.2023 CN 202310352819
(71) Applicant: Hangzhou Sciwind Biosciences Co., Ltd., Hangzhou, Zhejiang 310015 (CN); Sciwind Biosciences (Beijing) Co., Ltd., Beijing 100176 (CN)
(72) Inventor: LI, Yan, Beijing 100176 (CN); HAO, Sujuan, Beijing 100176 (CN); LI, Zhaoying, Beijing 100176 (CN); PAN, Hai, Beijing 100176 (CN)
(74) Representative: Snaith, James Michael
(86) International application number: PCT/CN2024/085852
(87) International publication number: WO 2024/208277

(57) **Abstract**

The present disclosure provides a calcitonin analogue and a derivative or a pharmaceutically acceptable salt thereof, a pharmaceutical composition comprising the analogue or the derivative or a pharmaceutically acceptable salt thereof and further comprising a pharmaceutically acceptable excipient, and use of the analogue, the derivative or a pharmaceutically acceptable salt thereof or the composition in the field of disease prevention/treatment.

## Description

### TECHNICAL FIELD

The present application relates to the technical field of polypeptides, specifically, a calcitonin analogue and a derivative and use thereof.

### BACKGROUND

Amylin, a polypeptide hormone consisting of 37 amino acids, is co-secreted with insulin in islet β cells and is lacking in patients with diabetes. It functions in several different organ systems primarily by virtue of amylin receptors 1-3 (AMYRs 1-3). Amylin can inhibit secretion of glucagon, delay gastric emptying, give a signal of satiety, and suppress appetite. Clinical studies have shown that amylin receptor agonists may be used for treating overweight, obesity, type 1 diabetes and/or type 2 diabetes. An amylin analogue known as Pramlintide (Trade name Symlin) may reduce the level of blood glucose and be used to treat patients having diabetes and taking insulin. However, Pramlintide has a half-life of less than an hour and is for mealtime administration, and thus a patient needs to take this medicine for treatment multiple times a day. Due to its instability and a pronounced disposition towards fibrillation at a neutral pH, which results in its precipitation and thus ineffectiveness, Pramlintide is provided in an acidic solution.

Calcitonin is a hormone produced in the thyroid gland that regulates levels of calcium and phosphates in the blood, and calcitonin receptors are found in many tissues throughout the body and involved in the regulation of bone remodeling. Salmon calcitonin currently marketed under the name of Miacalcic is useful in the treatment of diseases such as hypercalcemia and osteoporosis. Calcitonin is also unstable at a neutral pH and is thus provided in an acidic solution. Salmon calcitonin has a half-life of less than two hours and needs to be administered to a patient once or more per day. Calcitonin drugs currently available in the market are salmon calcitonin and elcatonin (eel calcitonin), which require daily administration by injection because of their low molecular weight. In addition, only exhibiting a desirable stability under acidic conditions but not under neutral ones poses a great barrier to the pharmaceutical use and formulation development of those molecule.

The short half-life of natural amylin and calcitonin receptor agonists has made prolongation of the half-life a target in developing such drugs. In addition, as a result of the poor stability and strong inclination to fibrillation of amylin and calcitonin receptor agonists at a neutral pH, improving stability at physiological pH has become another target in drug development.

### SUMMARY OF THE INVENTION

Accordingly, the present application makes improvements on agonistic polypeptides against amylin receptors so as to obtain analogues or their derivatives or pharmaceutically acceptable salts thereof, with an enhanced efficacy *in vivo* and an increased stability.

Specifically, the present application is directed to the following technical solution:
a derivative of calcitonin or a pharmaceutically acceptable salt thereof, comprising a polypeptide with an amino acid sequence of X₀ X₁SX₃LS TX₇VLG RLSX₁₄E LHRLX₂₀ X₂₁X₂₂PRT X₂₆X₂₇GX₂₉X₃₀ S X₃₂-NH₂,
wherein:
   X₀ is D or E, or is absent;
   X₁ is A, L, V, I, S, E, R or K;
   X₃ is Q, E or D;
   X₇ is A, L, V, I, S, R, E or D;
   X₁₄ is Q, D, E, Aib, S or T;
   X₂₀ is Q or E;
   X₂₁ is D or E;
   X₂₂ is Y, L or F;
   X₂₆ is D or Q;
   X₂₇ is V or S;
   X₂₉ is S or A;
   X₃₀ is G or E; and
   X₃₂ is P or trans-Hyp.

In a preferred embodiment, the derivative of calcitonin or a pharmaceutically acceptable salt thereof comprises a polypeptide with the following amino acid sequence: 
X0 X1SX3LS TX7VLG RLSX14E LHRLX20 X21X22PRT X26X27GX29X30 S X32-
NH2,
 wherein:
X₀ is D or E or is absent;
a combination of X₁ and X₇ is selected from A-A, L-L, V-V, I-I, S-S, A-S, S-A, E-R, R-E, R-D, K-E, K-D or E-E;
X₃ is Q, E or D;
X₁₄ is Q, D, E, Aib, S or T;
X₂₀ is Q or E;
X₂₁ is D or E;
X₂₂ is Y, L or F;
X₂₆ is D or Q;
X₂₇ is V or S;
a combination of X₂₉-X₃₀ is selected from S-G, A-G, A-E or S-E; and
X₃₂ is P or trans-Hyp.

In a preferred embodiment, X₀ is absent, the combination of X₁ and X₇ is A-A, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is L-L, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is V-V, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is I-I, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-S, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-S, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-A, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-R, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-E, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-D, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-E, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-D, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-E, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, X₃ is Q; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is L-L, X₃ is Q; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is V-V, X₃ is Q; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is I-I, X₃ is Q; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-S, X₃ is Q; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-S, X₃ is Q; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-A, X₃ is Q; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-R, X₃ is Q; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-E, X₃ is Q; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-D, X₃ is Q; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-E, X₃ is Q; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-D, X₃ is Q; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-E, X₃ is Q; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, or L-L, or V-V, or I-1, or S-S, or A-S, or S-A, or E-R, or R-E, or E-E, X₃ is D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, or L-L, or V-V, or I-I, or S-S, or A-S, or S-A, or E-R, or R-E, or E-E, X₃ is E; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, X₃ is Q, E or D; X₁₄ is Q, D or E; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is L-L, X₃ is Q, E or D; X₁₄ is Q, D or E; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is V-V, X₃ is Q, E or D; X₁₄ is Q, D or E; X₂₀ is Q or E; X₂₁ is D or E; X₂₂ is Y, L or F; X₂₆ is D or Q, X₂₇ is V or S, the combination of X₂₉-X₃₀ is selected from S-G, A-G, A-E or S-E; and X₃₂ is P or trans-Hyp.

In a preferred embodiment, X₀ is absent, the combination of X₁ and X₇ is I-I, X₃ is Q, E or D; X₁₄ is Q, D or E; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-S, X₃ is Q, E or D; X₁₄ is Q, D or E; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-S, X₃ is Q, E or D; X₁₄ is Q, D or E; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-A, X₃ is Q, E or D; X₁₄ is Q, D or E; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-R, X₃ is Q, E or D; X₁₄ is Q, D or E; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-E, X₃ is Q, E or D; X₁₄ is Q, D or E; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-D, X₃ is Q, E or D; X₁₄ is Q, D or E; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-E, X₃ is Q, E or D; X₁₄ is Q, D or E; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-D, X₃ is Q, E or D; X₁₄ is Q, D or E; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-E, X₃ is Q, E or D; X₁₄ is Q, D or E; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, or L-L, or V-V, or I-I, or S-S, or A-S, or S-A, or E-R, or R-E, or R-D, or K-E, or K-D or E-E, X₃ is Q, E or D; X₁₄ is Q; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, or L-L, or V-V, or I-I, or S-S, or A-S, or S-A, or E-R, or R-E, or R-D, or K-E, or K-D or E-E, X₃ is Q, E or D; X₁₄ is D; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, or L-L, or V-V, or I-I, or S-S, or A-S, or S-A, or E-R, or R-E, or R-D, or K-E, or K-D or E-E, X3 is Q, E or D; X₁₄ is E; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, or L-L, or V-V, or I-I, or S-S, or A-S, or S-A, or E-R, or R-E, or R-D, or K-E, or K-D or E-E, X3 is Q, E or D; X₁₄ is Aib; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, or L-L, or V-V, or I-I, or S-S, or A-S, or S-A, or E-R, or R-E, or R-D, or K-E, or K-D or E-E, X3 is Q, E or D; X₁₄ is S; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, or L-L, or V-V, or I-I, or S-S, or A-S, or S-A, or E-R, or R-E, or R-D, or K-E, or K-D or E-E, X₃ is Q, E or D; X₁₄ is T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, or L-L, or V-V, or I-I, or S-S, or A-S, or S-A, or E-R, or R-E, or R-D, or K-E, or K-D or E-E, X₃ is Q, E or D; X₁₄ is Q, D, E, Aib, S or T; X₂₀ is E; X₂₁ is D or E; X₂₂ is Y, L or F; and the combination of X₂₉-X₃₀ is selected from S-G, A-G, A-E or S-E.

In a preferred embodiment, X₀ is absent, the combination of X₁ and X₇ is A-A, X₃ is Q, E or D; X₁₄ is Q, D, E, Aib, S or T; X₂₀ is Q; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is L-L, X₃ is Q, E or D; X₁₄ is Q, D, E, Aib, S or T; X₂₀ is Q; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is V-V, X₃ is Q, E or D; X₁₄ is Q, D, E, Aib, S or T; X₂₀ is Q; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is I-I, X₃ is Q, E or D; X₁₄ is Q, D, E, Aib, S or T; X₂₀ is Q; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-S, X₃ is Q, E or D; X₁₄ is Q, D, E, Aib, S or T; X₂₀ is Q; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-S, X₃ is Q, E or D; X₁₄ is Q, D, E, Aib, S or T; X₂₀ is Q; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-A, X₃ is Q, E or D; X₁₄ is Q, D, E, Aib, S or T; X₂₀ is Q; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-R, X₃ is Q, E or D; X₁₄ is Q, D, E, Aib, S or T; X₂₀ is Q; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-E, X₃ is Q, E or D; X₁₄ is Q, D, E, Aib, S or T; X₂₀ is Q; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-D, X₃ is Q, E or D; X₁₄ is Q, D, E, Aib, S or T; X₂₀ is Q; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-E, X3 is Q, E or D; X₁₄ is Q, D, E, Aib, S or T; X₂₀ is Q; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-D, X₃ is Q, E or D; X₁₄ is Q, D, E, Aib, S or T; X₂₀ is Q; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-E, X₃ is Q, E or D; X₁₄ is Q, D, E, Aib, S or T; X₂₀ is Q; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, X₃ is Q, E or D; X₁₄ is Q, D, E, Aib, S or T; X₂₀ is Q or E; X₂₁ is E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is L-L, X₃ is Q, E or D; X₁₄ is Q, D, E, Aib, S or T; X₂₀ is Q or E; X₂₁ is E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is V-V, X₃ is Q, E or D; X₁₄ is Q, D, E, Aib, S or T; X₂₀ is Q or E; X₂₁ is E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is I-I, X₃ is Q, E or D; X₁₄ is Q, D, E, Aib, S or T; X₂₀ is Q or E; X₂₁ is E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-S, X₃ is Q, E or D; X₁₄ is Q, D, E, Aib, S or T; X₂₀ is Q or E; X₂₁ is E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-S, X₃ is Q, E or D; X₁₄ is Q, D, E, Aib, S or T; X₂₀ is Q or E; X₂₁ is E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-A, X₃ is Q, E or D; X₁₄ is Q, D, E, Aib, S or T; X₂₀ is Q or E; X₂₁ is E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-R, X₃ is Q, E or D; X₁₄ is Q, D, E, Aib, S or T; X₂₀ is Q or E; X₂₁ is E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-E, X₃ is Q, E or D; X₁₄ is Q, D, E, Aib, S or T; X₂₀ is Q or E; X₂₁ is E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-D, X₃ is Q, E or D; X₁₄ is Q, D, E, Aib, S or T; X₂₀ is Q or E; X₂₁ is E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-E, X₃ is Q, E or D; X₁₄ is Q, D, E, Aib, S or T; X₂₀ is Q or E; X₂₁ is E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-D, X₃ is Q, E or D; X₁₄ is Q, D, E, Aib, S or T; X₂₀ is Q or E; X₂₁ is E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-E, X₃ is Q, E or D; X₁₄ is Q, D, E, Aib, S or T; X₂₀ is Q or E; X₂₁ is E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, X₃ is Q, E or D; X₁₄ is Q, D, E, Aib, S or T; X₂₀ is Q or E; X₂₁ is D; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is L-L, X₃ is Q, E or D; X₁₄ is Q, D, E, Aib, S or T; X₂₀ is Q or E; X₂₁ is D; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is V-V, X₃ is Q, E or D; X₁₄ is Q, D, E, Aib, S or T; X₂₀ is Q or E; X₂₁ is D; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is I-I, X₃ is Q, E or D; X₁₄ is Q, D, E, Aib, S or T; X₂₀ is Q or E; X₂₁ is D; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-S, X₃ is Q, E or D; X₁₄ is Q, D, E, Aib, S or T; X₂₀ is Q or E; X₂₁ is D; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-S, X₃ is Q, E or D; X₁₄ is Q, D, E, Aib, S or T; X₂₀ is Q or E; X₂₁ is D; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-A, X₃ is Q, E or D; X₁₄ is Q, D, E, Aib, S or T; X₂₀ is Q or E; X₂₁ is D; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-R, X₃ is Q, E or D; X₁₄ is Q, D, E, Aib, S or T; X₂₀ is Q or E; X₂₁ is D; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-E, X₃ is Q, E or D; X₁₄ is Q, D, E, Aib, S or T; X₂₀ is Q or E; X₂₁ is D; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-D, X₃ is Q, E or D; X₁₄ is Q, D, E, Aib, S or T; X₂₀ is Q or E; X₂₁ is D; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-E, X₃ is Q, E or D; X₁₄ is Q, D, E, Aib, S or T; X₂₀ is Q or E; X₂₁ is D; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-D, X₃ is Q, E or D; X₁₄ is Q, D, E, Aib, S or T; X₂₀ is Q or E; X₂₁ is D; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-E, X₃ is Q, E or D; X₁₄ is Q, D, E, Aib, S or T; X₂₀ is Q or E; X₂₁ is D; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, X₃ is Q, E or D; X₁₄ is Q, D, E, Aib, S or T; X₂₀ is Q or E; X₂₁ is D or E; X₂₂ is Y; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is L-L, X₃ is Q, E or D; X₁₄ is Q, D, E, Aib, S or T; X₂₀ is Q or E; X₂₁ is D or E; X₂₂ is Y; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is V-V, X₃ is Q, E or D; X₁₄ is Q, D, E, Aib, S or T; X₂₀ is Q or E; X₂₁ is D or E; X₂₂ is Y; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is I-I, X₃ is Q, E or D; X₁₄ is Q, D, E, Aib, S or T; X₂₀ is Q or E; X₂₁ is D or E; X₂₂ is Y; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-S, X₃ is Q, E or D; X₁₄ is Q, D, E, Aib, S or T; X₂₀ is Q or E; X₂₁ is D or E; X₂₂ is Y; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-S, X₃ is Q, E or D; X₁₄ is Q, D, E, Aib, S or T; X₂₀ is Q or E; X₂₁ is D or E; X₂₂ is Y; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-A, X₃ is Q, E or D; X₁₄ is Q, D, E, Aib, S or T; X₂₀ is Q or E; X₂₁ is D or E; X₂₂ is Y; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-R, X₃ is Q, E or D; X₁₄ is Q, D, E, Aib, S or T; X₂₀ is Q or E; X₂₁ is D or E; X₂₂ is Y; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-E, X₃ is Q, E or D; X₁₄ is Q, D, E, Aib, S or T; X₂₀ is Q or E; X₂₁ is D or E; X₂₂ is Y; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-D, X₃ is Q, E or D; X₁₄ is Q, D, E, Aib, S or T; X₂₀ is Q or E; X₂₁ is D or E; X₂₂ is Y; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-E, X₃ is Q, E or D; X₁₄ is Q, D, E, Aib, S or T; X₂₀ is Q or E; X₂₁ is D or E; X₂₂ is Y; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-D, X₃ is Q, E or D; X₁₄ is Q, D, E, Aib, S or T; X₂₀ is Q or E; X₂₁ is D or E; X₂₂ is Y; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-E, X₃ is Q, E or D; X₁₄ is Q, D, E, Aib, S or T; X₂₀ is Q or E; X₂₁ is D or E; X₂₂ is Y; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, X₃ is Q, E or D; X₁₄ is Q, D, E, Aib, S or T; X₂₀ is Q or E; X₂₁ is D or E; X₂₂ is L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is L-L, X₃ is Q, E or D; X₁₄ is Q, D, E, Aib, S or T; X₂₀ is Q or E; X₂₁ is D or E; X₂₂ is L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is V-V, X₃ is Q, E or D; X₁₄ is Q, D, E, Aib, S or T; X₂₀ is Q or E; X₂₁ is D or E; X₂₂ is L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is I-I, X₃ is Q, E or D; X₁₄ is Q, D, E, Aib, S or T; X₂₀ is Q or E; X₂₁ is D or E; X₂₂ is L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-S, X₃ is Q, E or D; Xi₄ is Q, D, E, Aib, S or T; X₂₀ is Q or E; X₂₁ is D or E; X₂₂ is L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-S, X₃ is Q, E or D; X₁₄ is Q, D, E, Aib, S or T; X₂₀ is Q or E; X₂₁ is D or E; X₂₂ is L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is S-A, X₃ is Q, E or D; X₁₄ is Q, D, E, Aib, S or T; X₂₀ is Q or E; X₂₁ is D or E; X₂₂ is L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-R, X₃ is Q, E or D; X₁₄ is Q, D, E, Aib, S or T; X₂₀ is Q or E; X₂₁ is D or E; X₂₂ is L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-E, X₃ is Q, E or D; X₁₄ is Q, D, E, Aib, S or T; X₂₀ is Q or E; X₂₁ is D or E; X₂₂ is L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is R-D, X₃ is Q, E or D; X₁₄ is Q, D, E, Aib, S or T; X₂₀ is Q or E; X₂₁ is D or E; X₂₂ is L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-E, X₃ is Q, E or D; X₁₄ is Q, D, E, Aib, S or T; X₂₀ is Q or E; X₂₁ is D or E; X₂₂ is L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is K-D, X₃ is Q, E or D; X₁₄ is Q, D, E, Aib, S or T; X₂₀ is Q or E; X₂₁ is D or E; X₂₂ is L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent, the combination of X₁ and X₇ is E-E, X₃ is Q, E or D; X₁₄ is Q, D, E, Aib, S or T; X₂₀ is Q or E; X₂₁ is D or E; X₂₂ is L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent; the combination of X₁ and X₇ is selected from A-A, L-L, V-V, I-I, S-S, A-S, S-A, E-R, R-E or E-E; X₃ is Q or D; X₁₄ is Q, D, E, Aib, S or T; X₂₀ is Q or E; X₂₁ is D or E; X₂₂ is F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is absent; the combination of X₁ and X₇ is selected from A-A, L-L, V-V, I-I, S-S, A-S, S-A, E-R, R-E or E-E; X₃ is Q or D; X₁₄ is Q, D, E, Aib, S or T; X₂₀ is Q or E; X₂₁ is D or E; X₂₂ is Y, L or F; a combination of X₂₆-X₂₇ is D-V; the combination of X₂₉-X₃₀ is selected from S-G, A-G, A-E or S-E; and X₃₂ is P or trans-Hyp.

In a preferred embodiment, X₀ is absent; the combination of X₁ and X₇ is selected from A-A, L-L, V-V, I-I, S-S, A-S, S-A, E-R, R-E or E-E; X₃ is Q or D; X₁₄ is Q, D, E, Aib, S or T; X₂₀ is Q or E; X₂₁ is D or E; X₂₂ is Y, L or F; the combination of X₂₆-X₂₇ is Q-S; the combination of X₂₉-X₃₀ is selected from S-G, A-G, A-E or S-E; and X₃₂ is P or trans-Hyp.

In a preferred embodiment, X₀ is absent; the combination of X₁ and X₇ is selected from A-A, L-L, V-V, I-I, S-S, A-S, S-A, E-R, R-E or E-E; X₃ is Q or D; X₁₄ is Q, D, E, Aib, S or T; X₂₀ is Q or E; X₂₁ is D or E; X₂₂ is Y, L or F; the combination of X₂₆-X₂₇ is Q-V; the combination of X₂₉-X₃₀ is selected from S-G, A-G, A-E or S-E; and X₃₂ is P or trans-Hyp.

In a preferred embodiment, X₀ is absent; the combination of X₁ and X₇ is selected from A-A, L-L, V-V, I-I, S-S, A-S, S-A, E-R, R-E or E-E; X₃ is Q or D; X₁₄ is Q, D, E, Aib, S or T; X₂₀ is Q or E; X₂₁ is D or E; X₂₂ is Y, L or F; the combination of X₂₆-X₂₇ is D-S; the combination of X₂₉-X₃₀ is selected from S-G, A-G, A-E or S-E; and X₃₂ is P or trans-Hyp.

In a preferred embodiment, X₀ is absent, the combination of X₁ and X₇ is A-A, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is S-G; and X₃₂ is P or trans-Hyp.

In a preferred embodiment, X₀ is absent, the combination of X₁ and X₇ is L-L, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is S-G; and X₃₂ is P or trans-Hyp.

In a preferred embodiment, X₀ is absent, the combination of X₁ and X₇ is V-V, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is S-G; and X₃₂ is P or trans-Hyp.

In a preferred embodiment, X₀ is absent, the combination of X₁ and X₇ is I-I, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is S-G; and X₃₂ is P or trans-Hyp.

In a preferred embodiment, X₀ is absent, the combination of X₁ and X₇ is S-S, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is S-G; and X₃₂ is P or trans-Hyp.

In a preferred embodiment, X₀ is absent, the combination of X₁ and X₇ is A-S, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is S-G; and X₃₂ is P or trans-Hyp.

In a preferred embodiment, X₀ is absent, the combination of X₁ and X₇ is S-A, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is S-G; and X₃₂ is P or trans-Hyp.

In a preferred embodiment, X₀ is absent, the combination of X₁ and X₇ is E-R, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is S-G; and X₃₂ is P or trans-Hyp.

In a preferred embodiment, X₀ is absent, the combination of X₁ and X₇ is R-E, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is S-G; and X₃₂ is P or trans-Hyp.

In a preferred embodiment, X₀ is absent, the combination of X₁ and X₇ is R-D, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is S-G; and X₃₂ is P or trans-Hyp.

In a preferred embodiment, X₀ is absent, the combination of X₁ and X₇ is D-E, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is S-G; and X₃₂ is P or trans-Hyp.

In a preferred embodiment, X₀ is absent, the combination of X₁ and X₇ is K-D, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is S-G; and X₃₂ is P or trans-Hyp.

In a preferred embodiment, X₀ is absent, the combination of X₁ and X₇ is E-E, X₃ is Q, E or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is S-G; and X₃₂ is P or trans-Hyp.

In a preferred embodiment, X₀ is absent; the combination of X₁ and X₇ is selected from A-A, L-L, V-V, I-I, S-S, A-S, S-A, E-R, R-E or E-E; X₃ is Q or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is A-G; and X₃₂ is P or trans-Hyp.

In a preferred embodiment, X₀ is absent; the combination of X₁ and X₇ is selected from A-A, L-L, V-V, I-I, S-S, A-S, S-A, E-R, R-E or E-E; X₃ is Q or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is A-E; and X₃₂ is P or trans-Hyp.

In a preferred embodiment, X₀ is absent; the combination of X₁ and X₇ is selected from A-A, L-L, V-V, I-I, S-S, A-S, S-A, E-R, R-E or E-E; X₃ is Q or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is S-E; and X₃₂ is P or trans-Hyp.

In a preferred embodiment, X₀ is absent; the combination of X₁ and X₇ is selected from A-A, L-L, V-V, I-I, S-S, A-S, S-A, E-R, R-E or E-E; X₃ is Q or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P.

In a preferred embodiment, X₀ is absent; the combination of X₁ and X₇ is selected from A-A, L-L, V-V, I-I, S-S, A-S, S-A, E-R, R-E or E-E; X₃ is Q or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is trans-Hyp.

In a preferred embodiment, X₀ is D; the combination of X₁ and X₇ is selected from A-A, L-L, V-V, I-I, S-S, A-S, S-A, E-R, R-E or E-E; X₃ is Q or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is E; the combination of X₁ and X₇ is selected from A-A, L-L, V-V, I-I, S-S, A-S, S-A, E-R, R-E or E-E; X₃ is Q or D; X₁₄ is Q, D, E, Aib, S or T; X₂₀ is Q or E; X₂₁ is D or E; X₂₂ is Y, L or F; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is D or E or is absent, the combination of X₁ and X₇ is selected from A-A, L-L, V-V, I-I, S-S, A-S, S-A, R-E, R-D, K-E or K-D; X₃ is Q, E or D; X₁₄ is Q, D, E, Aib, S or T; X₂₀ is Q or E; X₂₁ is D or E; X₂₂ is Y or L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is D or E or is absent, the combination of X₁ and X₇ is selected from A-A, L-L, V-V, I-I, S-S, A-S, S-A, R-E, R-D, K-E or K-D; X₃ is Q, E or D; X₁₄ is Q, D, E, Aib, S or T; a combination of X₂₀-X₂₁ is E-E; X₂₂ is Y or L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is D or E or is absent, the combination of X₁ and X₇ is selected from A-A, L-L, V-V, I-I, S-S, A-S, S-A, R-E, R-D, K-E or K-D, X₃ is Q, E or D; a combination of X₁₄-X₂₂ is D-L; X₂₀ is Q or E; X₂₁ is D or E; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is D or E or is absent, the combination of X₁ and X₇ is selected from A-A, L-L, V-V, I-I, S-S, A-S, S-A, R-E, R-D, K-E or K-D, X₃ is Q, E or D; X₁₄ is Q, D, E, Aib, S or T; X₂₀ is Q or E; X₂₁ is D or E; X₂₂ is Y or L; X₂₆-X₂₇ is Q-S; the combination of X₂₉-X₃₀ is selected from S-G, A-G, A-E or S-E; and X₃₂ is P or trans-Hyp.

In a preferred embodiment, X₀ is D or E or is absent, the combination of X₁ and X₇ is selected from A-A, L-L, V-V, I-I, S-S, A-S, S-A, R-E, R-D, K-E or K-D, X₃-X₁₄-X₂₀ is E-E-E; X₂₁ is D or E; X₂₂ is Y or L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is D or E or is absent, the combination of X₁ and X₇ is selected from A-A, L-L, V-V, I-I, S-S, A-S, S-A, or R-E; X3 is Q, E or D; X₁₄ is Q, D, E, Aib, S or T; X₂₀ is Q or E; X₂₁ is D or E; X₂₂ is Y or L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is D or E or is absent, the combination of X₁ and X₇ is selected from A-A, L-L, V-V, I-I, S-S, A-S, S-A, or R-E; X₃ is Q, E or D; X₁₄ is Q, D, E, Aib, S or T; X₂₀-X₂₁ is E-E; X₂₂ is Y or L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is D or E or is absent, the combination of X₁ and X₇ is selected from A-A, L-L, V-V, I-I, S-S, A-S, S-A, or R-E; X₃ is Q, E or D; X₁₄-X₂₂ is D-L; X₂₀ is Q or E; X₂₁ is D or E; X₂₆ is D or Q; X₂₇ is V or S; the combination of X₂₉-X₃₀ is selected from S-G, A-G, A-E or S-E; and X₃₂ is P or trans-Hyp.

In a preferred embodiment, X₀ is D or E or is absent, the combination of X₁ and X₇ is selected from A-A, L-L, V-V, I-I, S-S, A-S, S-A, or R-E, X₃ is Q, E or D; X₁₄ is Q, D, E, Aib, S or T; X₂₀ is Q or E; X₂₁ is D or E; X₂₂ is Y or L; X₂₆-X₂₇ is Q-S; the combination of X₂₉-X₃₀ is selected from S-G, A-G, A-E or S-E; and X₃₂ is P or trans-Hyp.

In a preferred embodiment, X₀ is D or E or is absent, the combination of X₁ and X₇ is selected from A-A, L-L, V-V, I-I, S-S, A-S, S-A, or R-E, X₃-X₁₄-X₂₀ is E-E-E; X₂₁ is D or E; X₂₂ is Y or L; X₂₆ is D or Q; X₂₇ is V or S; the combination of X₂₉-X₃₀ is selected from S-G, A-G, A-E or S-E; and X₃₂ is P or trans-Hyp.

In a preferred embodiment, the derivative or a pharmaceutically acceptable salt thereof comprises a polypeptide with an amino acid sequence of ASQLS TAVLG RLSX₁₄E LHRLX₂₀ DX₂₂PRT DVGX₂₉G SP-NH₂, wherein,
X₁₄ is Q, D or E;
X₂₀ is Q or E, preferably Q;
X₂₂ is Y or L; and
X₂₉ is S or A.

In a preferred embodiment, the derivative or a pharmaceutically acceptable salt thereof comprises a polypeptide with an amino acid sequence of ASQLS TAVLG RLSX₁₄E LHRLQ DX₂₂PRT DVGSG SP-NH₂, wherein,
X₁₄ is Q, D or E, preferably Q or D; and
X₂₂ is Y or L.

In a preferred embodiment, the derivative of calcitonin or a pharmaceutically acceptable salt thereof comprises an amino acid sequence selected from any one of SEQ ID NO. 1-SEQ ID NO. 38 in Table 2.

In a preferred embodiment, the derivative of calcitonin or a pharmaceutically acceptable salt thereof comprises an amino acid sequence selected from any one of:
ASQLS TAVLG RLSDE LHRLQ DYPRT DVGSG SP-NH₂ (SEQ ID NO. 7),
ASQLS TAVLG RLSQE LHRLQ DLPRT DVGSG SP-NH₂ (SEQ ID NO. 17),
ASQLS TAVLG RLSEE LHRLQ DYPRT DVGSG SP-NH₂ (SEQ ID NO. 8),
ASQLS TAVLG RLSQE LHRLQ DYPRT DVGAG SP-NH₂ (SEQ ID NO. 18),
ASQLS TAVLG RLSQE LHRLQ DYPRT DVGAE SP-NH₂ (SEQ ID NO. 19) or
ASQLS TAVLG RLSQE LHRLE DYPRT DVGSG SP-NH₂ (SEQ ID NO. 12).

In a preferred embodiment, the derivative of calcitonin or a pharmaceutically acceptable salt thereof comprises a side chain, preferably a fatty acid-containing side chain, for modifying the amino acids.

In a preferred embodiment, the derivative of calcitonin or a pharmaceutically acceptable salt thereof comprises a fatty acid-containing side chain linked to the N terminus of the amino acid sequence.

In a preferred embodiment, the fatty acid-containing side chain is in the form of Z1+Z2+Z3, wherein Z1 is a C16-C22 fatty diacid;
Z2 is selected from any one of yGlu, αGlu, βAsp, αAsp, Inp, and Trx, or is absent; and
Z3 is n AEEA(s), wherein n≥0.
Z1,Z2, and Z3 are linked via amide linkages.
In a preferred embodiment, n is selected from 0, 1, 2, 3, 4, 5 or 6.

In a preferred embodiment, n is 6.

In a preferred embodiment, n is 4.

In a preferred embodiment, n is 2.

In a preferred embodiment, n is 0.

In a preferred embodiment, the fatty acid-containing side chain is in the form of (C16-C22 fatty diacid) + yGlu.

In a preferred embodiment, the fatty acid-containing side chain is in the form of (C16-C22 fatty diacid) + αGlu.

In a preferred embodiment, the fatty acid-containing side chain is in the form of (C16-C22 fatty diacid) + βAsp.

In a preferred embodiment, the fatty acid-containing side chain is in the form of (C16-C22 fatty diacid) + αAsp.

In a preferred embodiment, the fatty acid-containing side chain is in the form of (C16-C22 fatty diacid) + Inp.

In a preferred embodiment, the fatty acid-containing side chain is in the form of (C16-C22 fatty diacid) + Trx.

In a preferred embodiment, the fatty acid-containing side chain is in the form of (C16-C22 fatty diacid).

In a preferred embodiment, the fatty acid-containing side chain is in the form of (C16-C22 fatty diacid) + γGlu+2 AEEAs.

In a preferred embodiment, the fatty acid-containing side chain is in the form of (C16-C22 fatty diacid) + γGlu+4 AEEAs.

In a preferred embodiment, the fatty acid-containing side chain is in the form of (C16-C22 fatty diacid) + γGlu+6 AEEAs.

In a preferred embodiment, the fatty acid-containing side chain is in the form of (C16-C22 fatty diacid) + αGlu+2 AEEAs.

In a preferred embodiment, the fatty acid-containing side chain is in the form of (C16-C22 fatty diacid) + βAsp+2 AEEAs.

In a preferred embodiment, the fatty acid-containing side chain is in the form of (C16-C22 fatty diacid) + αAsp+2 AEEAs.

In a preferred embodiment, the fatty acid-containing side chain is in the form of (C16-C22 fatty diacid) + Inp+2 AEEAs.

In a preferred embodiment, the fatty acid-containing side chain is in the form of (C16-C22 fatty diacid) + Trx+2 AEEAs.

In a preferred embodiment, the fatty acid-containing side chain is in the form of (C20 fatty diacid) + yGlu.

In a preferred embodiment, the fatty acid-containing side chain is in the form of (C20 fatty diacid) + αGlu.

In a preferred embodiment, the fatty acid-containing side chain is in the form of (C20 fatty diacid) + βAsp.

In a preferred embodiment, the fatty acid-containing side chain is in the form of (C20 fatty diacid) + αAsp.

In a preferred embodiment, the fatty acid-containing side chain is in the form of (C20 fatty diacid) + Inp.

In a preferred embodiment, the fatty acid-containing side chain is in the form of (C20 fatty diacid) + Trx.

In a preferred embodiment, the fatty acid-containing side chain is in the form of (C20 fatty diacid).

In a preferred embodiment, the fatty acid-containing side chain is in the form of (C20 fatty diacid) + γGlu+2 AEEAs.

In a preferred embodiment, the fatty acid-containing side chain is in the form of (C20 fatty diacid) + γGlu+4 AEEAs.

In a preferred embodiment, the fatty acid-containing side chain is in the form of (C20 fatty diacid) + γGlu+6 AEEAs.

In a preferred embodiment, the fatty acid-containing side chain is in the form of (C20 fatty diacid) + αGlu+2 AEEAs.

In a preferred embodiment, the fatty acid-containing side chain is in the form of (C20 fatty diacid) + βAsp+2 AEEAs.

In a preferred embodiment, the fatty acid-containing side chain is in the form of (C20 fatty diacid)+ αAsp+2 AEEAs.

In a preferred embodiment, the fatty acid-containing side chain is in the form of (C20 fatty diacid) + Inp+2 AEEAs.

In a preferred embodiment, the fatty acid-containing side chain is in the form of (C20 fatty diacid) + Trx+2 AEEAs.

In a preferred embodiment, the fatty acid-containing side chain is in the form of (C22 fatty diacid) + γGlu+2 AEEAs.

In a preferred embodiment, the fatty acid-containing side chain is in the form of (C22 fatty diacid) + γGlu+4 AEEAs.

The present application also provides a polypeptide analogue, which is a polypeptide comprising an amino acid sequence of any one of the derivatives described above.

The present application also provides a pharmaceutical composition comprising one or more of the analogue, or the derivative or a pharmaceutically acceptable salt thereof described hereinabove. Preferably, the pharmaceutical composition further comprises a pharmaceutically acceptable excipient, e.g., a nontoxic filler, stabilizer, diluent, carrier, solvent, or the like.

The present application also provides use of the analogue, or the derivative or a pharmaceutically acceptable salt thereof described above in the manufacture of a medicament for preventing and/or treating overweight and/or obesity and/or type I or type II diabetes and/or osteoporosis and/or neuropathic pain.

The present application also provides use of the analogue, or the derivative or a pharmaceutically acceptable salt thereof described above in reducing food intake and/or body weight, for example, use thereof in the manufacture of a healthcare product, food, or medicament for reducing food intake and/or body weight.

The present application also provides a method of preventing and/or treating overweight and/or obesity and/or type I or type II diabetes and/or osteoporosis and/or neuropathic pain, comprising administering to a subject a prophylactically or therapeutically effective amount of the analogue, the derivative or a pharmaceutically acceptable salt thereof, or the pharmaceutical composition described hereinabove.

The present application also provides a method of reducing food intake, comprising administering to a subject an effective amount of the analogue, the derivative, or a pharmaceutically acceptable salt thereof, or the pharmaceutical composition described hereinabove.

### BRIEF DESCRIPTION OF THE DRAWINGS

The drawings are intended for a better understanding rather than any undue limitation of the present application, in which:
FIG. 1 is the spectrum of reversed phase chromatography of the molecule D3 (Mimylin) on Day 26 of a heat accelerated assay;
FIG. 2 is the spectrum of reversed phase chromatography of the calcitonin analogue M5 on Day 26 of a heat accelerated assay;
FIG. 3 is the spectrum of reversed phase chromatography of the molecule M8 alone on Day 26 of a heat accelerated assay under a neutral condition;
FIG. 4 is the spectrum of reversed phase chromatography of M8 and M51 in a neutral buffer solution without excipients after one month of a heat accelerated assay;
FIG. 5 is the spectrum of reversed phase chromatography of M8 and M51 in a neutral buffer solution with excipients after one month of a heat accelerated assay;
FIG. 6 is a trend chart showing the effect of the first group of calcitonin analogues or derivatives on weight change of rats in the experiment;
FIG. 7 is a trend chart showing the effect of the first group of calcitonin analogues or derivatives on accumulated food intake of rats in the experiment;
FIG. 8 is a trend chart showing the effect of the second group of calcitonin analogues or derivatives on weight change of rats in the experiment; and
FIG. 9 is a trend chart showing the effect of the second group of calcitonin analogues or derivatives on accumulated food intake of rats in the experiment.

### DETAILED DESCRIPTION

An "analogue" as described herein refers to a compound formed from a particular polypeptide molecule in which at least one amino acid residue has been substituted with another one, and/or at least one amino acid residue has been deleted, and/or at least one amino acid residue has been added to the N or C terminus, and/or at least one amino acid residue has been inserted between any two amino acids.

Terms "polypeptide" and "analogue", and the corresponding form thereof as used in a composition have the same meaning and are interchangeably used in the present application unless otherwise specified in the context. In certain cases, terms "calcitonin" and "calcitonin analogue" have the same meaning and are interchangeably used, for example in "a derivative of a calcitonin analogue" and "a derivative of calcitonin".

A "derivative" as described herein refers to a product resulting from a modification of a functional group (e.g., an amino acid residue) in a biomacromolecule (e.g., a polypeptide or protein) with certain groups or structures (e.g., structures of certain small molecules). The modification includes but is not limited to acylation, amidation, esterification, and thioesterification.

### Abbreviations

Corresponding names or structures of some abbreviations used in the examples of the present application are as follows:

| | | | |
|---|---|---|---|
| Trx: | 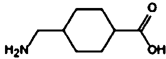 | AEEA: | 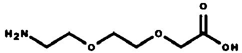 |
| Inp: | 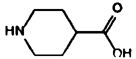 | | |

| | | | |
|---|---|---|---|
| AA: Amino Acid; Boc: t-Butyloxy carbonyl; DCM: dichloromethane; DMF: N,N-Dimethyl formamide; DIEA: N,N-Diisopropylethylamine; EDT: 1,2-Ethanedithiol; Fmoc: 9-fluorenylmethyloxycarbonyl; OtBu: t-butyl ester; Pbf: 2,2,4,6,7-Pentamethyldihydrobenzofuran-5-sulfonyl chloride; Pip: Piperidine; TBTU: O-(Benzotriazol-1-yl)-N,N,N',N',-tetramethyluronium Tetrafluoroborate; tBu: tertiary butyl; TFA: Trifluoroacetic acid; TIS: Triisopropylsilane; Trt: Triphenylmethyl; αGlu: α-glutamic acid; yGlu: γ-glutamic acid; αAsp: α-aspartic acid; and βAsp: β-aspartic acid. | | | |

Illustrative examples of the present application are described as below, in which various details of the examples in the present application are included for ease of understanding and should be construed as illustrative only. Therefore, it is to be realized by those of ordinary skill in the art that many alterations and modifications may be made to the examples described here without departing from the scope and spirit of the present application. For clarity and conciseness, the following description also omits depiction of well known functions and structures.

### EXAMPLES

### Example 1 Preparation of Polypeptide Derivatives

The polypeptides were synthesized by solid phase organic synthesis, specifically, solid-phase peptide synthesis (SPPS) with Fmoc-protected amino acids, and then cleaved, oxidized, and purified to yield target products. Taking the compound Cagrilintide (designated as "D1") as an example, the synthesis process was as below.

### 1.1 Solid-phase synthesis

Using Fmoc-Linker MBHA Resin S=0.32 mmol/g and employing Fmoc/tBu processing, amino acids were linked by sequential condensation from the C terminus to the N terminus (from right to left) as per the sequence of the aforesaid peptide in accordance with the method provided in Table 1.

**Table 1. Listing of synthesis procedures**

| **No.** | **Solvent** | **Number of times** | **duration (min/time)** |
|---|---|---|---|
| 1 | 20% Pip/DMF | 1 | 10 |
| 2 | 20% Pip/DMF | 1 | 5 |
| 3 | DMF | 5 | 1 |
| 4 | Sampling for colorimetric testing | | |
| 5 | AA/DIEA/TBTU | 1 | 60 |
| 6 | DMF | 4 | 1 |
| 7 | Sampling for colorimetric testing | | |
| Notes | 1. Repeating 1-7 till the end of the linear peptide synthesis | | |

The following amino acids were coupled in order:
A-01 Fmoc-Pro-OH, A-02 Fmoc-Thr(tBu)-OH, A-03 Fmoc-Asn(Trt)-OH, A-04 Fmoc-Ser(tBu)-OH, A-05 Fmoc-Gly-OH, A-06 Fmoc-Val-OH, A-07 Fmoc-Asn(Trt)-OH, A-08 Fmoc-Thr(tBu)-OH, A-09 Fmoc-Pro-OH, A-10 Fmoc-Pro-OH, A-11 Fmoc-Leu-OH, A-12 Fmoc-Ile-OH, A-13 Fmoc-Pro-OH, A-14 Fmoc-Gly-OH, A-15 Fmoc-Phe-OH, A-16 Fmoc-Asn(Trt)-OH, A-17 Fmoc-Asn(Trt)-OH, A-18 Fmoc-Ser(tBu)-OH, A-19 Fmoc-Ser(tBu)-OH, A-20 Fmoc-His(Trt)-OH, A-21 Fmoc-Arg(Pbf)-OH, A-22 Fmoc-Leu-OH, A-23 Fmoc-Phe-OH, A-24 Fmoc-Glu(OtBu)-OH, A-25 Fmoc-Ala-OH, A-26 Fmoc-Leu-OH, A-27 Fmoc-Arg(Pbf)-OH, A-28 Fmoc-Gln(Trt)-OH, A-29 Fmoc-Thr(tBu)-OH, A-30 Fmoc-Ala-OH, A-31 Fmoc-Cys(Trt)-OH, A-32 Fmoc-Thr(tBu)-OH, A-33 Fmoc-Ala-OH, A-34 Fmoc-Thr(tBu)-OH, A-35 Fmoc-Asn(Trt)-OH, A-36 Fmoc-Cys(Trt)-OH, A-37 Fmoc-Lys(Boc)-OH, A-38 Fmoc-Glu-otbu, and A-39 C20 diacid.

Finally, a polypeptide derivative-on-resin was formed.

The polypeptide derivative-on-resin was washed, transferred out, and dried to a constant weight for cleavage.

### 1.2 Cleavage

Formulation of a cleavage reagent: the cleavage reagent was TFA:H₂O:EDT:TIS = 95:1:2:2, the amount of which was a volume of 10 mL±2 mL per 1 g of the peptide-on-resin. H₂O, TFA, EDT, and TIS composing the required cleavage reagent were sequentially added to a cleavage reaction flask and were controlled at a temperature of 0-10°C. The cleavage reagent was added to the resin under stirring, and after the temperature of the system became stable, the reactants were stirred for another 2.5 hours under a controlled temperature of 25-30°C. The cleavage solution was filtered, and allowed for precipitation using glacial diethyl ether of a 5x volume of the liquid. The precipitates were filtered, washed 3 times with glacial diethyl ether of a 3x volume of the liquid, and then dried under reduced pressure at room temperature to yield a solid crude.

### 1.3 Oxidation

The crude was finely ground, and was slowly added, with stirring, to purified water, while an aqueous solution of acetonitrile was added dropwise. After the crude was added and completely dissolved, a methanol solution of iodine was added and stirred for half an hour.

### 1.4 Purification and lyophilization

The oxidated liquid as described above was filtered with a 0.45 um microporous filtering membrane. The crude was separated and purified using a column prepared by C-18 column packing material with a suitable gradient at ambient temperature, and then the target product was collected, detected and analyzed, and sorted. A purity ≥90% was required. Substandard target products were collected, and separated and purified again with a suitable gradient, to achieve qualified liquid peaks. The qualified liquid samples as mentioned above were lyophilized under reduced pressure to yield lyophilized powder of the refined polypeptide.

Polypeptides and derivatives prepared using a similar method to that described in Example 1 of the present application are shown in Table 2. D2 denotes the sequence of natural salmon calcitonin, D1 and D3 represent the sequences of modified calcitonins in the prior art, and the remaining are calcitonin analogues or derivatives provided in the present application, wherein the fatty diacid moiety of the polypeptide derivative is located at the outer side of the fatty acid-containing side chain for modification relative to the amino acid sequence, and thus is positioned at the distal end of the linkage of the side chain to the amino acid sequence.

**Table 2. Amino acid sequences and modifications**

| ID | SEQ ID NO. | Amino acid sequence | Site of modifica tion | Fatty acid-containing side chain |
|---|---|---|---|---|
| D1 | SEQ ID NO. 1 | KCNTATCATQ RLAEFLRHSS NNFGPILPPT NVGSNTP-NH2 | N terminus | C20 diacid + yGlu |
| D2 | SEQ ID NO. 2 | CSNLS TCVLG KLSQE LHKLQ TYPRT NTGSG TP-NH2 | None | None |
| D3 | SEQ ID NO. 3 | EASEL STAAL GRLSA ELHEL ATLPR TETGP ESP-NH2 | None | None |
| M5 | SEQ ID NO. 5 | ASQLS TAVLG RLSQE LHRLQ DYPRT DVGSG SP-NH2 | None | None |
| M6 | SEQ ID NO. 5 | ASQLS TAVLG RLSQE LHRLQ DYPRT DVGSG SP-NH2 | N terminus | C20 diacid + yGlu |
| M7 | SEQ ID NO. 6 | ASDLS TAVLG RLSQE LHRLQ DYPRT DVGSG SP-NH2 | N terminus | C20 diacid + yGlu |
| M8 | SEQ ID NO. 7 | ASQLS TAVLG RLSDE LHRLQ DYPRT DVGSG SP-NH2 | N terminus | C20 diacid + yGlu |
| M9 | SEQ ID NO. 8 | ASQLS TAVLG RLSEE LHRLQ DYPRT DVGSG SP-NH2 | N terminus | C20 diacid + yGlu |
| M10 | SEQ ID NO. 9 | ASQLS TAVLG RLS-Aib-E LHRLQ DYPRT DVGSG SP-NH2 | N terminus | C20 diacid + γGlu |
| M11 | SEQ ID NO. 10 | ASQLS TAVLG RLSSE LHRLQ DYPRT DVGSG SP-NH2 | N terminus | C20 diacid + yGlu |
| M12 | SEQ ID NO. 11 | ASQLS TAVLG RLSTE LHRLQ DYPRT DVGSG SP-NH2 | N terminus | C20 diacid + yGlu |
| M13 | SEQ ID NO. 12 | ASQLS TAVLG RLSQE LHRLE DYPRT DVGSG SP-NH2 | N terminus | C20 diacid + yGlu |
| M14 | SEQ ID NO. 13 | ASQLS TAVLG RLSQE LHRLE EYPRT DVGSG SP-NH₂ | N terminus | C20 diacid + yGlu |
| M15 | SEQ ID NO. 14 | ASQLS TAVLG RLSQE LHRLQ DYPRT QVGSG SP-NH₂ | N terminus | C20 diacid + γGlu |
| M16 | SEQ ID NO. 15 | ASQLS TAVLG RLSQE LHRLQ DYPRT DSGSG SP-NH2 | N terminus | C20 diacid + γGlu |
| M17 | SEQ ID NO. 16 | ASQLS TAVLG RLSQE LHRLQ DYPRT QSGSG SP-NH2 | N terminus | C20 diacid + yGlu |
| M18 | SEQ ID NO. 17 | ASQLS TAVLG RLSQE LHRLQ DLPRT DVGSG SP-NH2 | N terminus | C20 diacid + yGlu |
| M19 | SEQ ID NO. 18 | ASQLS TAVLG RLSQE LHRLQ DYPRT DVGAG SP-NH2 | N terminus | C20 diacid + yGlu |
| M20 | SEQ ID NO. 19 | ASQLS TAVLG RLSQE LHRLQ DYPRT DVGAE SP-NH2 | N terminus | C20 diacid + yGlu |
| M21 | SEQ ID NO. 20 | ASQLS TAVLG RLSQE LHRLQ DYPRT DVGSE SP-NH2 | N terminus | C20 diacid + yGlu |
| M22 | SEQ ID NO. 21 | ASQLS TAVLG RLSQE LHRLQ DYPRT DVGSG S-trans-Hyp -NH2 | N terminus | C20 diacid + yGlu |
| M23 | SEQ ID NO. 22 | D ASQLS TAVLG RLSQE LHRLQ DYPRT DVGSG SP-NH₂ | N terminus | C18 diacid |
| M24 | SEQ ID NO. 23 | E ASQLS TAVLG RLSQE LHRLQ DYPRT DVGSG SP-NH2 | N terminus | C18 diacid |
| M25 | SEQ ID NO. 24 | LSQLS TLVLG RLSQE LHRLQ DYPRT DVGSG SP-NH2 | N terminus | C20 diacid + yGlu |
| M26 | SEQ ID NO. 25 | VSQLS TVVLG RLSQE LHRLQ DYPRT DVGSG SP-NH2 | N terminus | C20 diacid + yGlu |
| M27 | SEQ ID NO. 26 | ISQLS TIVLG RLSQE LHRLQ DYPRT DVGSG SP-NH2 | N terminus | C20 diacid + yGlu |
| M28 | SEQ ID NO. 27 | SSQLS TSVLG RLSQE LHRLQ DYPRT DVGSG SP-NH2 | N terminus | C20 diacid + yGlu |
| M29 | SEQ ID NO. 28 | ASQLS TSVLG RLSQE LHRLQ DYPRT DVGSG SP-NH2 | N terminus | C20 diacid + γGlu |
| M30 | SEQ ID NO. 29 | SSQLS TAVLG RLSQE LHRLQ DYPRT DVGSG SP-NH2 | N terminus | C20 diacid + γGlu |
| M31 | SEQ ID NO. 30 | ESQLS TRVLG RLSQE LHRLQ DYPRT DVGSG SP-NH₂ | N terminus | C20 diacid + γGlu |
| M32 | SEQ ID NO. 31 | RSQLS TEVLG RLSQE LHRLQ DYPRT DVGSG SP-NH2 | N terminus | C20 diacid + γGlu |
| M33 | SEQ ID NO. 32 | ESQLS TEVLG RLSQE LHRLQ DYPRT DVGSG SP-NH₂ | N terminus | C20 diacid + γGlu |
| M34 | SEQ ID NO. 33 | ASELS TAVLG RLSEE LHRLE DYPRT DVGSG SP-NH2 | N terminus | C20 diacid + γGlu |
| M35 | SEQ ID NO. 34 | RSQLS TDVLG RLSQE LHRLQ DYPRT DVGSG SP-NH2 | N terminus | C20 diacid+γGlu |
| M36 | SEQ ID NO. 35 | KSQLS TEVLG RLSQE LHRLQ DYPRT DVGSG SP-NH2 | N terminus | C20 diacid+γGlu |
| M37 | SEQ ID NO. 36 | KSQLS TDVLG RLSQE LHRLQ DYPRT DVGSG SP-NH2 | N terminus | C20 diacid+γGlu |
| M38 | SEQ ID NO. 7 | ASQLS TAVLG RLSDE LHRLQ DYPRT DVGSG SP-NH₂ | N terminus | C22 diacid + yGlu |
| M39 | SEQ ID NO. 7 | ASQLS TAVLG RLSDE LHRLQ DYPRT DVGSG SP-NH2 | N terminus | C18 diacid + yGlu |
| M40 | SEQ ID NO. 7 | ASQLS TAVLG RLSDE LHRLQ DYPRT DVGSG SP-NH2 | N terminus | C16 diacid + yGlu |
| M41 | SEQ ID NO. 7 | ASQLS TAVLG RLSDE LHRLQ DYPRT DVGSG SP-NH₂ | N terminus | C20 diacid + αGlu |
| M42 | SEQ ID NO. 7 | ASQLS TAVLG RLSDE LHRLQ DYPRT DVGSG SP-NH2 | N terminus | C18 diacid + aGlu |
| M43 | SEQ ID NO. 7 | ASQLS TAVLG RLSDE LHRLQ DYPRT DVGSG SP-NH₂ | N terminus | C20 diacid + βAsp |
| M44 | SEQ ID NO. 7 | ASQLS TAVLG RLSDE LHRLQ DYPRT DVGSG SP-NH2 | N terminus | C18 diacid + αAsp |
| M45 | SEQ ID NO. 7 | ASQLS TAVLG RLSDE LHRLQ DYPRT DVGSG SP-NH2 | N terminus | C20 diacid + Trx |
| M46 | SEQ ID NO. 7 | ASQLS TAVLG RLSDE LHRLQ DYPRT DVGSG SP-NH2 | N terminus | C18 diacid + Trx |
| M47 | SEQ ID NO. 7 | ASQLS TAVLG RLSDE LHRLQ DYPRT DVGSG SP-NH₂ | N terminus | C20 diacid + Inp |
| M48 | SEQ ID NO. 7 | ASQLS TAVLG RLSDE LHRLQ DYPRT DVGSG SP-NH2 | N terminus | C18 diacid + Inp |
| M49 | SEQ ID NO. 37 | ASQLS TAVLG RLSQE LHRLQ DFPRT DVGSG SP-NH2 | N terminus | C20 diacid+γGlu |
| M50 | SEQ ID NO. 38 | ASQLS TAVLG RLSDE LHRLQ DLPRT DVGSG SP-NH2 | N terminus | C20 diacid+γGlu |
| M51 | SEQ ID NO. 4 | HVEGTFTSDVSSYLEEQAAREFI KWLVRGRG-OH | 30K | C18 diacid+γGlu +2 AEEAs |
| M52 | SEQ ID NO. 8 | ASQLS TAVLG RLSEE LHRLQ DYPRT DVGSG SP-NH2 | N terminus | C20 diacid+αGlu |
| M53 | SEQ ID NO. 8 | ASQLS TAVLG RLSEE LHRLQ DYPRT DVGSG SP-NH2 | N terminus | C20 diacid+βAsp |
| M54 | SEQ ID NO. 8 | ASQLS TAVLG RLSEE LHRLQ DYPRT DVGSG SP-NH2 | N terminus | C20 diacid+αAsp |
| M55 | SEQ ID NO. 8 | ASQLS TAVLG RLSEE LHRLQ DYPRT DVGSG SP-NH2 | N terminus | C20 diacid + Trx |
| M56 | SEQ ID NO. 8 | ASQLS TAVLG RLSEE LHRLQ DYPRT DVGSG SP-NH2 | N terminus | C20 diacid + Inp |
| M57 | SEQ ID NO. 8 | ASQLS TAVLG RLSEE LHRLQ DYPRT DVGSG SP-NH2 | N terminus | C20 diacid+γGlu+2 AEEAs |
| M58 | SEQ ID NO. 8 | ASQLS TAVLG RLSEE LHRLQ DYPRT DVGSG SP-NH2 | N terminus | C20 diacid + αGlu+2 AEEAs |
| M59 | SEQ ID NO. 8 | ASQLS TAVLG RLSEE LHRLQ DYPRT DVGSG SP-NH2 | N terminus | C20 diacid + βAsp+2 AEEAs |
| M60 | SEQ ID NO. 8 | ASQLS TAVLG RLSEE LHRLQ DYPRT DVGSG SP-NH₂ | N terminus | C20 diacid + αAsp+2 AEEAs |
| M61 | SEQ ID NO. 8 | ASQLS TAVLG RLSEE LHRLQ DYPRT DVGSG SP-NH2 | N terminus | C20 diacid + Trx+2 AEEAs |
| M62 | SEQ ID NO. 8 | ASQLS TAVLG RLSEE LHRLQ DYPRT DVGSG SP-NH₂ | N terminus | C20 diacid + Inp+2 AEEAs |
| M63 | SEQ ID NO. 8 | ASQLS TAVLG RLSEE LHRLQ DYPRT DVGSG SP-NH2 | N terminus | C20 diacid + γGlu+4 AEEAs |
| M64 | SEQ ID NO. 8 | ASQLS TAVLG RLSEE LHRLQ DYPRT DVGSG SP-NH2 | N terminus | C20 diacid + γGlu+6 AEEAs |
| M65 | SEQ ID NO. 8 | ASQLS TAVLG RLSEE LHRLQ DYPRT DVGSG SP-NH₂ | N terminus | C22 diacid+γGlu |
| M66 | SEQ ID NO. 8 | ASQLS TAVLG RLSEE LHRLQ DYPRT DVGSG SP-NH₂ | N terminus | C22 diacid + γGlu+2 AEEAs |
| M67 | SEQ ID NO. 8 | ASQLS TAVLG RLSEE LHRLQ DYPRT DVGSG SP-NH2 | N terminus | C22 diacid + γGlu+4 AEEAs |

### Example 2 Testing for Activity of the Derivatives in Cells

The objective of this assay is to test the activity or potency of calcitonin analogues or derivatives on human amylin receptors *in vitro* using a luciferase assay.

### 2.1 Construction of an amylin receptor/CRE-luc cell line

Using the standard protocol, CHO-K1/Gal5/AMY3 cells (purchased from GenScript, with the calcitonin receptor and the receptor activity modifying peptide (RAMP) constructed already) were transfected with a plasmid containing a luciferase expression cassette driven by multiple copies of cAMP response elements (CREs). The cells were cultured in an F12 medium containing 200 ug/mL Zeocin, 2 ug/mL puromycin, 100 ug/mL hygromycin, and 400 ug/mL G418 to afford a stably transfected amylin receptor/CRE-luc cell line.

### 2.2 Luciferase assay of amylin

The lyophilized powder obtained according to the method of Example 1 was dissolved in 20 mM phosphate buffer solution with a pH of 7.0, and diluted in a growth medium (F12 culture medium containing 10% FBS) to give a derivative sample with an initial concentration of 10 nM, which was then subjected to a 5-fold serial dilution in the growth medium to afford samples of 7 concentrations including 10 nM, 2 nM, 0.4 nM, etc. To each well of a white 96-well plate was added 50 µL of the tested sample solution at a respective concentration.

The stably transfected amylin receptor/CRE-luc CHO cells were resuspended in the growth medium at a certain density, and 50 µL of the cell suspension was added at a density of about 20000 cells per well to the white 96-well plate containing the tested sample solution. After incubation of 24 hours at 37°C and 5% CO₂, 100 µL of a Luciferase substrate was added to each well, incubated for 3 minutes, and finally was tested for luminescence on SpectraMax L (Molecular Devices) with the software SoftMax Pro 7.0.3 GxP. A standard curve was plotted using the fluorescent intensities for calculation of EC50. Results were shown in Table 3 as below.

**Table 3. Results for activity against amylin receptors in cells**

| ID | EC50/nM | ID | EC50/nM | ID | EC50/nM | ID | EC50/nM |
|---|---|---|---|---|---|---|---|
| D1 | 0.036 | M19 | 0.016 | M36 | 0.032 | M53 | 0.017 |
| D4 | 0.031 | M20 | 0.017 | M37 | 0.052 | M54 | 0.017 |
| M5 | 0.033 | M21 | 0.017 | M38 | 0.022 | M55 | 0.017 |
| M6 | 0.017 | M22 | 0.017 | M39 | 0.016 | M56 | 0.017 |
| M8 | 0.017 | M23 | 0.010 | M40 | 0.002 | M57 | 0.017 |
| M9 | 0.017 | M24 | 0.016 | M41 | 0.069 | M58 | 0.017 |
| M10 | 0.016 | M25 | 0.016 | M42 | 0.016 | M59 | 0.017 |
| M11 | 0.017 | M26 | 0.079 | M43 | 0.017 | M60 | 0.016 |
| M12 | 0.017 | M27 | 0.016 | M44 | 0.016 | M61 | 0.015 |
| M13 | 0.017 | M28 | 0.017 | M46 | 0.119 | M62 | 0.016 |
| M14 | 0.017 | M29 | 0.017 | M47 | 0.082 | M63 | 0.037 |
| M15 | 0.016 | M30 | 0.017 | M48 | 0.063 | M64 | 0.044 |
| M16 | 0.025 | M32 | 0.030 | M49 | 0.075 | M65 | 0.017 |
| M17 | 0.042 | M34 | 0.058 | M50 | 0.017 | M66 | 0.017 |
| M18 | 0.028 | M35 | 0.037 | M52 | 0.017 | M67 | 0.017 |

The data in the table demonstrated that most of the calcitonin analogues or derivatives provided in the present application had strong activity against amylin receptors in cells, and some of the derivatives even had an activity value that was one order of magnitude higher than calcitonin analogues or derivatives in the prior art.

### Example 3 Heat Accelerated Stability Assay

### 3.1 Materials and devices

A stability test chamber (BINDER GmbH), a 0.01 mg-readability balance (METTLER TOLEDO), a pH meter (METTLER TOLEDO), a biosafety cabinet (ESCO), T2G-II small-sized motor-driven capping machine (Changsha zhongya pharmaceutical equipment co. LTD), a high-speed refrigerated centrifuge (Eppendorf), a sterilizer cabinet (Shinva Medical), Agilent 1260 high-performance liquid chromatographer, and Sepax Bio-C18 4.6*250 mm 3 µm 200 Å reversed phase column were used.

Reagents and consumables included ultrapure water (18.2 MΩ, self-made), acetonitrile (HPLC grade), trifluoroacetic acid (HPLC grade), sodium dihydrogen phosphate (pharmaceutical grade, Hunan Jiudian Hongyang Pharmaceutical Co., Ltd), hydrochloric acid (pharmaceutical grade, Hunan Er-Kang Pharmaceutical Co., Ltd), sodium hydroxide (Analytical Reagent grade, Hushi^{®}, Sinopharm Chemical Reagent Co., Ltd), vials, rubber stoppers, disposable syringes, and sterile filters.

### 3.2 Methods of the assay

The lyophilized powder (1 mg/mL) of calcitonin analogues or derivatives prepared in accordance with the method of Example 1 were mixed with sodium dihydrogen phosphate (1.42 mg/mL) and dissolved in ultrapure water in the indicated mass-volume ratios. After adjusting pH to about 7.4 with hydrochloric acid/sodium hydroxide, the solution was filtered into a sterilized vial in a clean bench using a 0.22 µm sterile filter. The vial was then capped and placed into the stability test chamber at 40°C. Detections were made on Days 7 (7d) and 26 (26d), and during the assay, changes in the characteristics and properties of the polypeptides were observed and recorded. The sample was then centrifuged for 3 min at 10000 rpm at 4°C, and the supernatant was transferred to a liquid phase sample vial for detecting the concentration and purity of the polypeptides using liquid phase chromatography described as below, wherein the concentration of the polypeptide = peak area/injection volume/extinction coefficient/60×flow rate, and the purity of the polypeptide = target peak area/total peak area× 100%.

Conditions of the reversed phase chromatography Flow rate: 1.0 ml/min; Autosampler temperature: 15°C; Column temperature: 25°C; Detection wavelengths: 280 nm and 214 nm; Mobile phase A: 100% H₂O+0.05% TFA; Mobile phase B: 100% ACN; and Gradient of elution listed in Table 4:

**Table 4. Listing of the gradient of elution in the liquid phase chromatography**

| | | | | | | |
|---|---|---|---|---|---|---|
| Time (min) | 0 | 3 | 8 | 13 | 14 | 20 |
| B% | 25 | 25 | 70 | 70 | 25 | 25 |

### 3.3 Results of the assay

Results were shown below in Tables 5, 6, and 7. A difference of more than 5% in content as compared with the initial value was typically assumed (for example, as prescribed by the *Chinese Pharmacopeia (2020 Edition)*) to be a significant change. Within a reasonable margin of error, a decrease of less than 1% from the initial value to the detected content is denoted as "no change" below.

**Table 5. Changes in characteristics or properties in the heat accelerated stability assay**

| ID | 0d | 7d | ID | 0d | 7d | ID | 0d | 7d |
|---|---|---|---|---|---|---|---|---|
| D1 | Clear | Clear | M16 | Clear | Clear | M40 | Clear | Clear |
| D2 | Clear | Clear | M19 | Clear | Clear | M41 | Clear | Clear |
| D3 | Clear | Clear | M22 | Clear | Clear | M42 | Clear | Clear |
| M5 | Clear | Clear | M24 | Clear | Clear | M43 | Clear | Clear |
| M7 | Clear | Clear | M27 | Clear | Clear | M44 | Clear | Clear |
| M8 | Clear | Clear | M29 | Clear | Clear | M45 | Clear | Clear |
| M9 | Clear | Clear | M32 | Clear | Clear | M46 | Clear | Clear |
| M10 | Clear | Clear | M34 | Clear | Clear | M47 | Clear | Clear |
| M11 | Clear | Clear | M38 | Clear | Clear | M48 | Clear | Clear |
| M13 | Clear | Clear | M39 | Clear | Clear | | | |

As shown in Table 5, the calcitonin analogues or derivatives provided in the present application had no noticeable change in characteristics or properties during the heat accelerated stability assay under a neutral condition. The solution was still clear on Day 7 of the accelerated testing, indicating almost no characteristic or property change of the polypeptides. Molecules such as M8, M9 and M13 were further found to exhibit no significant change in characteristics or properties and remain to be in a clear solution even on Day 26 of the accelerated testing, manifesting considerably stable characteristics and properties.

**Table 6. Concentration changes in the heat accelerated stability assay**

| ID | Concentration change (7d-0d)/% | ID | Concentration change (7d-0d)/% | ID | Concentration change (7d-0d)/% |
|---|---|---|---|---|---|
| D2 | -40.064 | M22 | No change | M41 | -3.134 |
| M5 | -1.051 | M24 | No change | M42 | -3.344 |
| M7 | No change | M27 | No change | M43 | No change |
| M8 | No change | M29 | No change | M44 | -2.285 |
| M9 | No change | M32 | No change | M45 | -2.670 |
| M10 | No change | M34 | -1.618 | M46 | No change |
| M11 | No change | M4 | No change | M47 | -3.488 |
| M13 | No change | M38 | No change | M48 | -1.678 |
| M16 | No change | M39 | No change | | |
| M19 | No change | M40 | -2.280 | | |

Table 6 showed that there was no obvious change in concentration of the calcitonin analogues or derivatives provided in the present application during the heat accelerated stability assay under a neutral condition. The concentration changes were all below 4% on Day 7 of the accelerated testing, and were found to be close to 1% (e.g., M5) or even below 1% (denoted as "no change"; e.g., M8 and M13) for some of the calcitonin analogues or derivatives. M8 and M13 had a concentration change of less than 2% even on Day 26 of the accelerated testing as compared to Day 0, exhibiting a high stability in concentration.

**Table 7. Purity changes in the heat accelerated stability assay (Purity from the reversed phase chromatography-214 nm)**

| ID | Change% (7d-0d) | Change% (26d-0d) | ID | Change% (7d-0d) | Change% (26d-0d) |
|---|---|---|---|---|---|
| D1 | -12.73 | / | M32 | No change | / |
| D2 | -47.82 | / | M34 | No change | / |
| D3 | -6.85 | -18.00 | M4 | No change | No change |
| M5 | No change | No change | M8 | No change | -2.21 |
| M7 | No change | No change | M38 | No change | No change |
| M8 | No change | No change | M39 | No change | -3.3 |
| M9 | No change | No change | M40 | -1.67 | -4.82 |
| M10 | No change | / | M41 | -1.66 | -5.98 |
| M11 | No change | / | M42 | No change | -2.93 |
| M13 | No change | No change | M43 | No change | -1.96 |
| M16 | No change | / | M44 | -1.15 | -3.76 |
| M19 | No change | / | M45 | No change | -1.77 |
| M22 | No change | / | M46 | No change | -5.67 |
| M24 | No change | / | M47 | No change | No change |
| M27 | No change | / | M48 | -1 | -3.9 |
| M29 | No change | | | | |

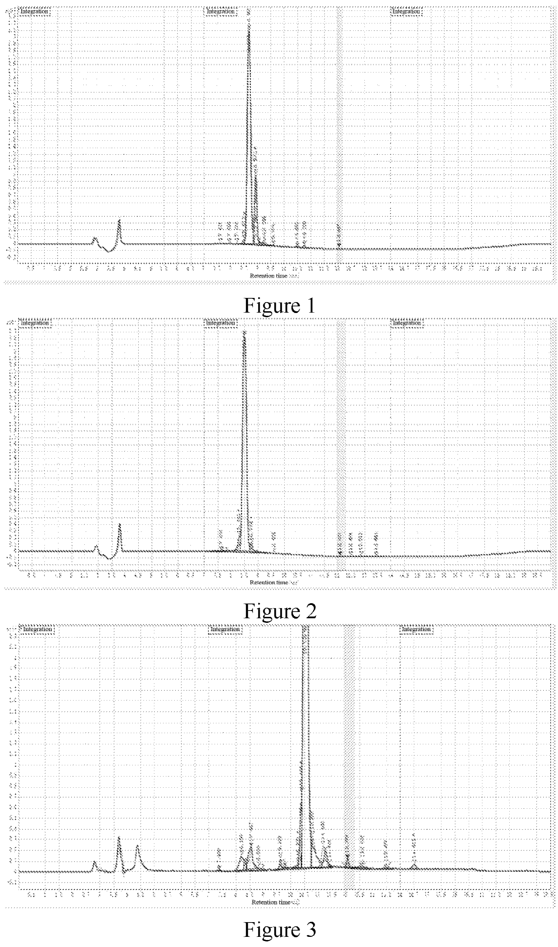

Table 7 revealed that there was no significant change in purity of the calcitonin analogues or derivatives provided in the present application during the heat accelerated stability assay under a neutral condition. On Day 7 of the accelerated testing, purity changes of most of the polypeptide solutions were less than 1% as determined by the reversed phase chromatography, or even no purity decrease was observed; and molecules such as M5, M8, M9 and M13 were further found to have a lower change or even nearly no noticeable change in purity on Day 26 of the accelerated testing, showing great stability in purity. In contrast to a purity decrease of 18% in the molecule D3 in the prior art on Day 26 of the heat accelerated assay (see FIG. 1), the purity of the calcitonin analogue M5 of the present application was decreased by only 0.68% at the same timepoint (see FIG. 2). A comparison in purity changes of the calcitonin derivative M8 provided in the present application and D1 (Cagrilintide, Novo Nordisk) in the heat accelerated assay was shown in Table 8, and the spectra of reversed phase chromatography of the molecule M8 prior to and after the heat accelerated assay were shown in FIG. 3. The difference between the backbones of M8 and D1 was clearly shown by the data from Table 8 and FIG. 3, and the former had an excellent stability under a neutral condition.

**Table 8. Comparison of results from liquid phase analysis on 26d of the heat accelerated assay**

| Molecular ID | Reversed phase chromatography | |
|---|---|---|
| | 7d change | 26d change |
| D1 | -12.73% | / |
| M8 | No change | -0.12% |

As indicated by the data of the present application, the molecules of calcitonin analogues and derivatives provided herein had significantly higher stability in the heat accelerated assay than those in the prior art.

### Example 4 Heat Accelerated Stability Assay for Compatibility Testing

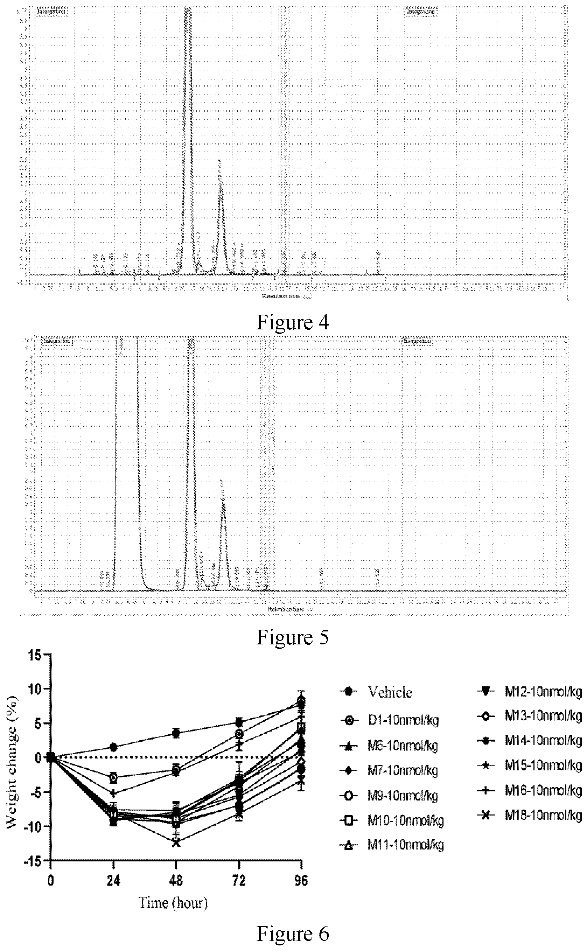

The calcitonin analogue M8 (1 mg/ml), the GLP-1 receptor agonist M51 (1 mg/ml), and sodium dihydrogen phosphate (1.42 mg/ml) were mixed and dissolved in ultrapure water, and 5.5 mg/ml phenol and 14 mg/ml 1,2-dihydroxypropane were further added for the testing group with excipients. After pH was adjusted to about 7.4 with hydrochloric acid/sodium hydroxide, accelerated stability assays were performed according to the method of Example 3. The results were shown in FIG. 4 (without excipients) and FIG. 5 (with excipients), which demonstrated that M8 was well compatible with M51, showing only a little change in the spectra of reversed phase chromatography after a one-month heat accelerated assay under the same formulation condition with M51. Thus, they had the potential to be developed as a co-formulation. As shown in the results of the present heat accelerated assays, the calcitonin analogues or derivatives provided herein exhibited remarkably good stability, for example the molecule M8, which has excellent thermal stability both as a single drug and when combined with M51 under the same formulation condition.

### Example 5 Study of Weight Change and Food Consumption in Animals

SD (Sprague Dawley) rats weighing 200-250 g were used for this study. The rats were received at least 10-14 days before the study began such that they could accommodate to the experimental environment. After arrival, they were exposed to a reversed light/dark cycle (i.e., lights switched off in daytime and on in nighttime) for two weeks and were individually housed for the first week so as to ensure high data accuracy and test sensitivity. Throughout the acclimation and experiment period, the rats had free access to food and water. 5-8 rats were assigned to each testing group for the derivatives and were subcutaneously administered the derivatives at a dose of 10 nmol/kg or a vehicle, and the dosing timepoint was recorded for each group. After dosing, the rats were put back into cages where they were housed and could have access to food and water. Food consumption and weight change of the rats were recorded online or manually every 24 hours.

The first and second groups of experimental results were shown in FIGs. 6 and 7 and FIGs. 8 and 9 respectively, which suggested that the calcitonin analogues and derivatives of the present application produced an appreciable effect on reducing body weight and food intake of the rats.

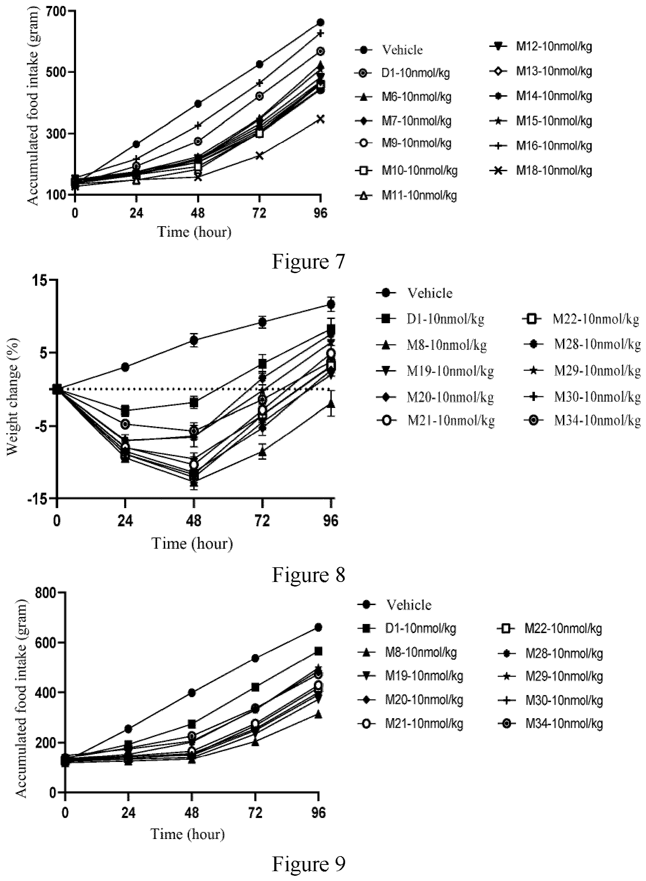

FIGs. 6 and 7 further revealed that the calcitonin analogues of the present application had a superior effect to D1 in terms of weight loss and appetite suppression, among which M18, M9 and M13 were particularly effective.

FIGs. 8 and 9 further revealed that the calcitonin analogues and derivatives of the present application such as M8, M19 and M20 had a prominent effect on both reducing body weight and food intake of the rats.

Although embodiments of the present application have been described as above, the present application is not limited to the above specific embodiments, which are only illustrative and directory but not restrictive, and application fields. Without departing from the scope of the claims of the present application, many variants may occur to those of ordinary skill in the art with inspiration from this specification.

## Claims

1. A derivative of calcitonin or a pharmaceutically acceptable salt thereof, comprising a polypeptide with an amino acid sequence of X₀ X₁SX₃LS TX₇VLG RLSX₁₄E LHRLX₂₀ X₂₁X₂₂PRT X₂₆X₂₇GX₂₉X₃₀ S X₃₂-NH₂,
wherein:
X₀ is D or E, or is absent;
X₁ is A, L, V, I, S, E, R or K;
X₃ is Q, E or D;
X₇ is A, L, V, I, S, R, E or D;
X₁₄ is Q, D, E, Aib, S or T;
X₂₀ is Q or E;
X₂₁ is D or E;
X₂₂ is Y, L or F;
X₂₆ is D or Q;
X₂₇ is V or S;
X₂₉ is S or A;
X₃₀ is G or E; and
X₃₂ is P or trans-Hyp.

2. The derivative or a pharmaceutically acceptable salt thereof of claim 1, wherein a combination of X₁ and X₇ is selected from A-A, L-L, V-V, I-I, S-S, A-S, S-A, E-R, R-E, R-D, K-E, K-D or E-E; and a combination of X₂₉-X₃₀ is selected from S-E, S-G, A-G or A-E.

3. The derivative or a pharmaceutically acceptable salt thereof of claim 1 or 2, wherein X₃ is Q.

4. The derivative or a pharmaceutically acceptable salt thereof of any one of claims 1-3, wherein X₁₄ is Q, D or E.

5. The derivative or a pharmaceutically acceptable salt thereof of any one of claims 1-4, wherein X₂₀ is Q.

6. The derivative or a pharmaceutically acceptable salt thereof of any one of claims 1-5, wherein X₂₁ is D.

7. The derivative or a pharmaceutically acceptable salt thereof of any one of claims 1-6, wherein X₂₂ is Y or L.

8. The derivative or a pharmaceutically acceptable salt thereof of any one of claims 1-7, wherein a combination of X₂₆ and X₂₇ is selected from D-V, Q-S, Q-V, and D-S.

9. The derivative or a pharmaceutically acceptable salt thereof of any one of claims 1-8, wherein the combination of X₂₉-X₃₀ is S-G.

10. The derivative or a pharmaceutically acceptable salt thereof of any one of claims 1-9, wherein X₃₂ is P.

11. The derivative or a pharmaceutically acceptable salt thereof of any one of claims 1-4, comprising a polypeptide with an amino acid sequence of ASQLS TAVLG RLSX₁₄E LHRLX₂₀ DX₂₂PRT DVGX₂₉G SP-NH₂, wherein,
X₁₄ is Q, D or E;
X₂₀ is Q or E, preferably Q;
X₂₂ is Y or L; and
X₂₉ is S or A.

12. The derivative or a pharmaceutically acceptable salt thereof of claim 11, comprising a polypeptide with an amino acid sequence of ASQLS TAVLG RLSX₁₄E LHRLQ DX₂₂PRT DVGSG SP-NH₂, wherein,
X₁₄ is Q, D or E, preferably Q or D; and
X₂₂ is Y or L.

13. The derivative or a pharmaceutically acceptable salt thereof of claim 1, wherein the amino acid sequence is selected from any one of SEQ ID NO. 1-SEQ ID NO. 38.

14. The derivative or a pharmaceutically acceptable salt thereof of any one of claims 1-8 or 10-13, wherein the sequence of the polypeptide is any one of:
ASQLS TAVLG RLSDE LHRLQ DYPRT DVGSG SP-NH₂,
ASQLS TAVLG RLSQE LHRLQ DLPRT DVGSG SP-NH₂,
ASQLS TAVLG RLSEE LHRLQ DYPRT DVGSG SP-NH₂,
ASQLS TAVLG RLSQE LHRLQ DYPRT DVGAG SP-NH₂,
ASQLS TAVLG RLSQE LHRLQ DYPRT DVGAE SP-NH₂, or
ASQLS TAVLG RLSQE LHRLE DYPRT DVGSG SP-NH₂.

15. The derivative or a pharmaceutically acceptable salt thereof of any one of claims 1-14, wherein the derivative comprises a fatty acid-containing side chain linked to the N terminus of the polypeptide.

16. The derivative or a pharmaceutically acceptable salt thereof of claim 15, wherein the fatty acid-containing side chain is in the form of Z1+Z2+Z3, wherein Z1 is a C16-C22 fatty diacid;
Z2 is selected from any one of yGlu, αGlu, βAsp, αAsp, Inp, and Trx, or is absent;
Z3 is n AEEA(s), wherein n≥0; and
Z1, Z2, and Z3 are linked via amide linkages.

17. The derivative or a pharmaceutically acceptable salt thereof of claim 16, wherein Z1 is a C20 fatty diacid.

18. The derivative or a pharmaceutically acceptable salt thereof of claim 16 or 17, wherein Z2 is yGlu.

19. The derivative or a pharmaceutically acceptable salt thereof of any one of claims 16-18, wherein n is selected from 0, 1, 2, 3, 4, 5 or 6.

20. A polypeptide comprising an amino acid sequence of the derivative of any one of claims 1-14.

21. A pharmaceutical composition comprising the derivative of any one of claims 1-19 or the polypeptide of claim 20, or a pharmaceutically acceptable salt thereof, and a pharmaceutically acceptable excipient.

22. Use of the derivative of any one of claims 1-19 or the polypeptide of claim 20, or a pharmaceutically acceptable salt thereof, in the manufacture of a medicament for preventing and/or treating overweight and/or obesity and/or type I or type II diabetes and/or osteoporosis and/or neuropathic pain.

23. Use of the derivative of any one of claims 1-19 or the polypeptide of claim 20, or a pharmaceutically acceptable salt thereof, in reducing food intake.

24. A method of preventing and/or treating overweight and/or obesity and/or type I or type II diabetes and/or osteoporosis and/or neuropathic pain, comprising administering to a subject a prophylactically or therapeutically effective amount of the derivative of any one of claims 1-19 or the polypeptide of claim 20, or a pharmaceutically acceptable salt thereof, or the pharmaceutical composition of claim 21.

25. A method of reducing food intake, comprising administering to a subject an effective amount of the derivative of any one of claims 1-19 or the polypeptide of claim 20, or a pharmaceutically acceptable salt thereof, or the pharmaceutical composition of claim 21.
